# EUROPEAN PATENT APPLICATION

(11) **EP 2 253 294 A1**
(43) Date of publication of application: **24.11.2010**
(21) Application number: 10163625.6
(22) Date of filing: 21.05.2010
(51) Int. Cl.: A61F 13/00

(54) **Absorptive foam gel lock wound dressing**

(30) Priority: 21.05.2009 US 216904 P
(71) Applicant: Euromed Inc., Orangeburg NY 10962 (US); Jensen, Jarl, Orangeburg NY 10962 (US); Ramjit, Ravi, Orangeburg NY 10962 (US)
(72) Inventor: Jensen, Jarl, Orangeburg, NY 10962 (US); Ramjit, Ravi, Orangeburg, NY 10962 (US)
(74) Representative: Bender, Mikkel

(57) **Abstract**

The present invention relates to a wound dressing comprising a hydrophilic foam layer; a hydrocolloid layer wherein said hydrocolloid layer is contoured with one or more pockets (e.g. at least one surface of the hydro colloid layer, preferably the distal one, is 3-dimensionally structured by, for example, one or more protrusions); and a film backing layer. Said pockets increase the absorptive capacity of the said dressing.

## Description

The present invention relates to a wound dressing with an improved exudate absorption capacity and to a process of manufacturing such a wound dressing.

### BACKGROUND OF THE INVENTION

Treating heavily draining wounds is a problem because the wound fluid leaks out of the fluid soaked dressing and is deposited on the healthy skin causing maceration, slowing the healing process. Thus, in wound care, one of the main objectives of a wound dressing is to increase, improve or maximize utilization of the absorbent capacity of the dressing so as to reduce or eliminate maceration, and facilitate the healing process of the wound. The control of exudates is of prime importance if a moist wound microenvironment is to be maintained.

Unfortunately, many wound dressings have been found to remove all the exudates that a wound produces, thereby causing a "dry" wound that is undesirable in the wound healing process or in the alternative, such wound dressings have been found to absorb or control the exudates insufficiently, thereby leading to a pooling of the exudates which may increase the risk of bacterial proliferation and lead to infection.

Currently available wound dressings have an absorbent layer containing hydrophilic materials that absorb exudates and permit the wound dressing to be left in place for a period of days. Such absorbent layers may comprise a non-woven material or foam containing hydrocolloid particles or hydrophilic foams.

While absorbent layer dressings are configured to absorb wound exudates, they often possess the disadvantage of being limited in the amount of exudates that may be absorbed. The limit to the maximum absorption of absorbent foam is often directly related to their geometrical size prior to absorbing a fluid. For example, hydrophilic foams may expand only to 12-15% of their original size. Another disadvantage is that a certain amount of the exudate can be "squeezed" out of absorbent foam dressings due to poor liquid retention. The ability of exudates to be squeezed from the foam layer, and, thus, from the dressing itself, poses a risk of infection and may interfere with the healing of the wound.

Yet another disadvantage with known dressings is that absorption of exudate by an absorptive layer in contact with the wound causes the central portion of the applied dressing to swell and push up against the wound. Continued swelling can induce separation of the skin adherent layer from the skin outside the wound area; especially at the border of the wound dressing where a "curling" effect may occur. This excessive swelling of the wound dressing may lead to further leakage of the exudate from the periphery of the dressing, thereby providing a tract for the invasion of pathogenic microorganisms and further promoting maceration of the wound site.

Conventionally, a backing layer is provided that comprises a liquid impervious film that is attached to the absorbent layer to prevent exudate from seeping from the dressing. A difficulty arises during fluid uptake in that as the absorbent core expands, the backing layer must accommodate the expansion of the absorbent layer without causing curling of the dressing.

Those skilled in the art have been working to solve these problems. For example, U.S. Patent No. 4,738,257 discloses a backing layer formed of a thin elastic sheet which is yieldable as the absorbent core swells. It has been found, however, that a liquid impervious plastic film cannot be made to sufficiently stretch in keeping with the expansion of the absorbent layer, and as a result, the film counteracting the swelling absorbent layer may produce the aforesaid curling at the border of the dressing. Another proposed solution is provided in U.S. Patent No. 6,040,492 which discloses a wound dressing that includes a backing layer that is attached to an absorbent foam core and includes a plurality of wrinkles that substantially flatten as the foam core swells. While the backing layer may accommodate the expansion of the foam core, the fluid uptake of this wound dressing is limited by the expandability of the foam core itself. Accordingly, due to the limited absorptive capacity of the foam core, the dressing must be replaced often.

Most recently, U.S. Patent Application No. 2008/0255493 discloses a wound dressing comprising an absorbent core with a plurality or array of receptacles arranged in a predetermined pattern. The depth of each receptacle is generally 4-5 mm, and positioned at least 0.5 mm from the facing layer. In an embodiment of US 2008/0255493, the pattern may be tailored to include more receptacles at specific regions of the dressing as opposed to other regions. Said receptacles extend a distance into the absorbent core, just short of its entire thickness. The receptacles may assume a variety of configurations and may be cylindrical in shape, extend transversely along at least a portion of the distal surface of the absorbent core, or assume other possible configurations. The plurality of receptacles contain discrete portions of absorbent material that absorb exudate from the wound and migrate from the receptacles towards the backing layer upon absorption of such exudate.

US 2008/0255493 discloses useful foams such as polyurethane, cellulose, carboxylated butadiene-styrene rubber, polyester foams, hydrophilic epoxy foams or polyacrylate. In a preferred embodiment, the foam is formed from hydrophilic polyurethane foam. The absorbent material described in the US 2008/0255493 disclosure is comprised of superabsorbent polymeric granulates, flakes or powders that swell on exposure to water and form a hydrated gel (hydrogel) by absorbing large amounts of water. These superabsorbent materials generally fall into three classes, namely starch graft copolymers, cross-linked carboxymethylcellulose derivatives and modified hydrophilic polyacrylates.

One drawback of the US 2008/0255493 dresssing is the fact that the backing layer, which initially is attached to the distal surface of the absorbent core, detaches from said surface due to migration of absorbent material upon wound fluid uptake. This may lead to undesirable instability of the backing layer. Furthermore, the fact that the absorbent material, which expands and migrates upon liquid contact, is arranged in receptacles within the absorbent core leads to inevitable compression of the latter in areas lying in between two receptacles. This may result in undesirable loss of absorbent capacity and liquid conductivity of the absorbent core. In addition, the design of the US 2008/0255493 dressing is fairly complex, which renders its manufacture tedious and time-consuming. First, a plurality of receptacles has to be formed in the absorbent core, for example by using a plurality of heated projection elements. Next, a silicone film is place between aligned top and bottom plates having a plurality of holes that correspond to, and have to be aligned with, the receptacles. Finally, a predetermined amount of absorbent material is injected into each receptacle followed by attachment of the backing layer.

Hence, there is still a need in the art for wound dressings with the increased absorptive capacity to manage heavily exudating wounds, thus controlling skin maceration.

It is therefore a first object of the present invention to provide a wound dressing that is designed to absorb the wound fluid from these heavily exudating wounds while eliminating the problem of leakage onto healthy skin.

It is a second object of the present invention to provide a wound dressing that is easily manufactured and simple to handle.

Not wishing to be bound by theory, Applicant's invention creates additional absorptive capacity over currently available dressings by contouring the hydrocolloid layer with one or more pockets. In other words, said contouring is accomplished by embossing or stamping said hydrocolloid layer with pockets. The pattern of these pockets can be random or organized. There can be one pocket or a plurality of pockets. The pockets can be of any size or shape (i.e. geometry) and said pockets may vary in depth. The pockets lock in the wound fluid ( i.e. the exudate)

Further, the pockets may contain active ingredients. Thus, the present invention is useful because of its exudate absorption capacity and because said invention can deliver active ingredients to a wound site.

The new and unique way in which one or more of the above-mentioned objects are achieved is to provide a wound dressing comprising a hydrophilic foam layer (a), a hydrocolloid layer (b), and a film backing layer (d), wherein said hydrocolloid layer (b) is contoured with one or more pockets (c).

In another aspect, the present invention relates to a method of manufacturing a wound dressing of the present invention, said method comprising the steps of:
- providing a hydrophilic foam layer (a) and a hydrocolloid layer (b),
- coating the hydrophilic foam layer (a) with the hydrocolloid layer (b) to form an absorbent layer, and
- embossing or stamping the absorbent layer with a pattern, thereby creating one or more pockets.

### Definitions and usages of terms

As used herein, the term "pockets" refers to the shapes that are in discontinuous patterns throughout the absorbent layer. As used herein, a synonym for a pocket may be a well or a protrusion, depending on the perspective of the viewer.

As used herein, the term "Absorbent layer" refers to the hydrophilic foam layer (a) and the hydrocolloid layer (b).

### "Exudate" means wound fluid.

"Maceration" means healthy skin deterioration due to exposure of wound fluid or excessive moisture.

"Contouring" means shaping or forming. Said shaping may be accomplished by means including, but not limited to, embossing and stamping. In the present invention, contouring means forming the pockets and shaping the dressing. A hydrocolloid layer "contoured" with one or more pockets means that at least one surface of the hydrocolloid layer, preferably the distal one, is 3-dimensionally structured by, for example, one or more protrusions.

The terms "pressing", "stamping", and "embossing" may be used interchangeably.

As used herein, the term "proximal" refers to a location within the wound dressing that, when the wound dressing is applied to a wound, is closer to the wound surface than a location termed "distal". Each layer of the inventive wound dressing has opposing proximal and distal surfaces, wherein the proximal surface is closer to the wound than the distal surface when in use. A given distal surface may also be termed "back" of said layer.

As used herein, the "thickness" of a given layer is the shortest distance between its proximal and distal surface. Hence, where a layer has a non-constant thickness, e.g. because of the provision of pockets, the thickness refers to the thickness of the areas with no pockets, which is the shortest distance between proximal and distal surface of the layer. The "depth" of a pocket refers to the height of the protrusion, i.e. the vertical distance between the top of the protrusion and the surrounding surface within which the protrusion is formed.

As used herein, the term "geometrically ordered" refers to an ordered arrangement of pockets where each pocket has an equal and constant distance to neighbouring pockets.

### BRIEF DESCRIPTION OF THE DRAWINGS

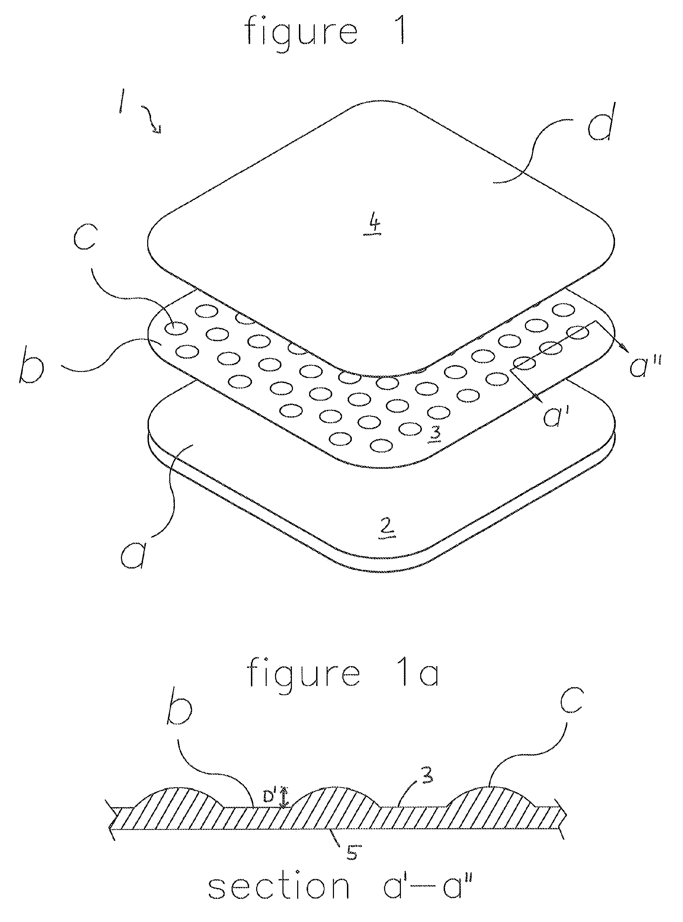

Figure 1 is an exploded view of the wound dressing of the present invention.
Figure 1 a is an enlarged sectional view taken along section a'-----a" of Figure 1.
Figure 2 is a plan view of the wound dressing of the present invention.
Figure 3 shows test results comparing the inventive wound dressing with known products.

### DETAILED DESCRIPTION OF THE INVENTION

The present invention relates to a wound dressing comprising a hydrophilic foam layer (a), a hydrocolloid layer (b), and a film backing layer (d), wherein said hydrocolloid layer (b) is contoured with one or more pockets (c). The hydrophilic foam layer comprises one or more hydrophilic foams as described below. The hydrocolloid layer comprises one or more hydrocolloids as described below. The film backing layer is preferably liquid impervious and vapor permeable.

According to a preferred embodiment of the present invention, the proximal surface of the hydrocolloid layer (b) is attached to the distal surface of the hydrophilic foam layer (a), and wherein the one or more pockets (c) are formed as protrusions formed within the distal surface of the hydrocolloid layer (b). Such a dressing thus comprises a hydrophilic foam layer that is in direct contact with the wound surface when in use. Liquid emanating from the wound is absorbed and transported by the hydrophilic foam layer towards the hydrocolloid layer. The latter has a greater thickness in the areas of the pockets as compared to areas where no pockets are formed. The hydrocolloid layer absorbs the liquid from the hydrophilic foam layer. As a result of this, the hydrocolloid layer, in particular its pockets, may swell and/or form a gel. Due to the inventive pocket design this will, however, not result in detachment of the film backing layer since the inter-pocket space on the distal surface of the hydrocolloid layer offers ample space for pocket expansion.

According to another embodiment, the pockets, i.e. the protrusions, may take the form of spherical segments.

Advantageously, the film backing layer is attached directly to the hydrocolloid layer.

### Hydrophilic Foams

Hydrophilic Foams useful in the practice of the present invention include, but are not limited to, commercially available medical grade foams, such as polyurethane. In another embodiment of the invention the polyurethane foam is preferred.

### Hydrocolloids

Hydrocolloids are known in the art as useful medical adhesives. Hydrocolloids useful in the practice of the present invention include, but are not limited to, water absorbing and/or water swellable material such as carboxymethylcellulose, pectin, gelatin, high molecular weight carbowax, carboxypolymethylene, carboxymethyl starches, alginates, carrageenan, gelatine, citrus pectin, powdered pectin, synthetic or natural gums, such as gum guar, gum arabic, locust bean gum, karaya and mixtures thereof. In an embodiment of the invention, the preferred hydrocolloids are carboxymethylcellulose, alginates and pectin. In yet another embodiment of the invention, any number of hydrocolloids can be blended together. In another embodiment of the invention, carboxymethylcellulose is preferred.

In an embodiment of the invention, said hydrocolloid layer may optionally contain absorbent polymers including, but not limited to, commercially available medical grade absorbent polymers such as polyacrylates, polyacrylamides, polypropylenes, and newer polymers incorporating biological molecules such as graft copolymers of sodium acrylate and 1-vinyl-2-pyrrolidone polymerized with carboxy methyl chitosan.

### Films

Films useful in preparing a backing layer include, but are not limited to, commercially available medical grade polyurethane, PVC, Polypropylene, and olefin films. In an embodiment of the invention polyurethane is preferred.

According to one embodiment of the present invention, the pockets are arranged in a geometrically ordered fashion.

According to a preferred embodiment, the wound dressing consists of the hydrophilic foam layer (a), the hydrocolloid layer (b), and the film backing layer (d), wherein said hydrocolloid layer (b) is contoured with one or more pockets (c). Hence, no other layers such as a silicone facing layer are present in the wound dressing. The improved absorptive capacity of the inventive pocket arrangement makes this simple design of the wound dressing possible. A wound-dressing of this embodiement is simple to produce, cost-efficient and easy to handle.

According to one embodiment of the present invention, at least 20% of the distal surface of the hydrocolloid layer is made up of pockets. This ensures a superior absorptive capacity.

In another aspect, the present invention relates to a method of manufacturing a wound dressing of the present invention, said method comprising the steps of:
- providing a hydrophilic foam layer (a) and a hydrocolloid layer (b),
- coating the hydrophilic foam layer (a) with the hydrocolloid layer (b) to form an absorbent layer, and
- embossing or stamping the absorbent layer with a pattern, thereby creating one or more pockets.

This method is simple and cost-effective. It results in a wound dressing product with improved absorptive capacity and decreased leakage of wound fluid from the dressing. The present invention also relates to any products obtainable by said method.

According to a preferred embodiment of the inventive method, the coating step is carried out on the distal surface of the hydrophilic foam layer (a).

According to a particularly preferred embodiment of the inventive method the one or more pockets are formed as protrusions formed within the distal surface of the hydrocolloid layer.

According to another embodiment of the inventive method a film backing layer is attached to the distal surface of the hydrocolloid layer prior to the embossing or stamping step. The film backing layer is preferably made of polyurethane.

According to a preferred embodiment of the inventive method, the hydrocolloid layer comprises an active ingredient or a mixture thereof.

Figure 1 is an exploded view illustration of the present invention showing the inventive wound dressing 1. In the Figure 1 embodiment of the invention, the Hydrophilic foam layer (a) is coated with a Hydrocolloid layer (b). The hydrophilic foam layer (a) and the hydrocolloid layer (b) form the absorbent layer. Then, this absorbent layer, (a) plus (b), is embossed or stamped with a desired pattern of shapes thereby creating the absorptive pockets (c). In other words, during the embossing process, portions of the hydrocolloid layer (b) are pressed into the hydrophilic foam layer (a) to create the one or more absorptive pockets (c). The pockets (c) may vary in depth. The geometry of the pockets (c) may vary. In other words, said pockets (c) may be any shape, including but not limited to, circular, triangular or diamond shaped. The depth of the pocket (c) can be as shallow or as deep as desired. The only requirement is that said pocket (c) not penetrate through to the other side of the dressing. A polyurethane film backing (d) is applied to one side of the dressing. In an embodiment, said polyurethane film (d) backing is applied to the absorbent layer. Said absorbent layer is (a) plus (b).

Figure 1 shows also distal surface 2 of the hydrophilic foam layer (a), distal surface 3 of the hydrocolloid layer (b), and distal surface 4 of the film backing layer. The proximal surface 5 of the hydrocolloid layer (b) is shown in Figure 1a.

Figure 1 a is an enlarged sectional view taken along section a'-----a" of Figure 1. Specifically, the Figure 1a view is a sectional view of the pocket (c) created in the hydrocolloid layer (b) during the embossing process.

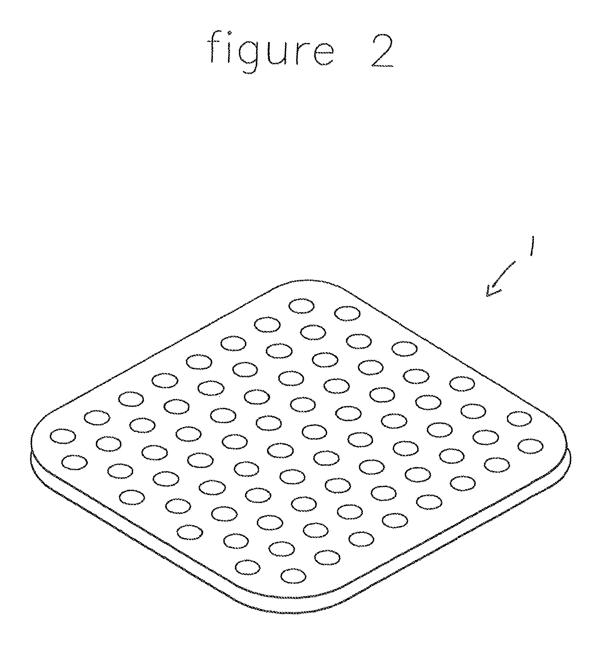

Figure 2 is a plan view of wound dressing of the present invention as it may be applied to a wound.

In another aspect of the invention, active ingredients may be contained within said one or more the pockets. Active ingredients useful in the practice of the present invention include, but are not limited to, pharmaceutically active substances belonging to one or more active ingredient classes such as ACE inhibitors, adrenergics, adrenocorticosteroids, acne therapeutic agents, aldose reductase inhibitors, aldosterone antagonists, alpha-glucosidase inhibitors, alpha-1-antagonists, remedies for alcohol abuse, amino acids, amebicides, anabolics, analeptics, anesthetic additions, anesthetics (non-inhalational), anesthetics (local), analgesics, androgens, angina therapeutic agents, antagonists, antiallergics, antiallergics for asthma treatment, further antiallergics (e.g. leukotriene antagonists, antianemics, antiandrogens, antianxiolytics, anti-arthritics, antiarrhythmics, antiatheriosclerotics, antibiotics, anticholinergics, anticonvulsants, anti-depressants, antidiabetics, antidiarrheals, anti-diuretics, antidotes, antiemetics, antiepileptics, antifibrinolytics, antiepileptics, antihelmintics, antihistamines, antihypotensives, antihypertensives, antihypertensives, antihypotensives, anticoagulants, antimycotics, antiestrogens, antiestrogens (non-steroidal), antiparkinson agents, antiinflammatory agents, antiproliferative active ingredients, antiprotozoal active ingredients, antirheumatics, antischistosomicides, antispasmolytics, antithrombotics, antitussives, antivirals, appetite suppressants, arteriosclerosis remedies, bacteriostatics, beta-blockers, beta-receptor blockers, bronchodilators, carbonic anhydrase inhibitors, chemotherapeutic agents, choleretics, cholinergics, cholinergic agonists, cholinesterase inhibitors, agents for the treatment of ulcerative colitis, diuretics, ectoparasiticides, emetics, enzymes, enzyme inhibitors, enzyme inhibitors, active ingredients to counter vomiting, fibrinolytics, fungistatics, gout remedies, glaucoma therapeutic agents, glucocorticoids, glucocorticosteroids, hemostatics, cardiac glycosides, histamine H2 antagonists, hormones and their inhibitors, immunotherapeutic agents, cardiotonics, coccidiostats, laxatives, lipid-lowering agents, gastrointestinal therapeutic agents, malaria therapeutic agents, migraine remedies, microbiocides, Crohn's disease remedies, metastasis inhibitors, migraine remedies, mineral preparations, motility-increasing active ingredients, muscle relaxants, neuroleptics, active ingredients for treatment of estrogens, osteoporosis, otologicals, antiparkinson agents, phytopharmaceuticals, proton pump inhibitors, prostaglandins, active ingredients for treating benign prostate hyperplasia, active ingredients for treating pruritus, psoriasis active ingredients, psychoactive drugs, radical scavengers, renin antagonists, thyroid therapeutic agents, active ingredients for treating seborrhea, active ingredients to counter seasickness, spasmolytics, alpha- and beta-sympathomimetics, platelet aggregation inhibitors, tranquilizers, ulcer therapeutic agents, agents for the treatment of urolithiasis, vitamins, cytokines, active ingredients for combination therapy with cytostatics, cytostatics and mixtures thereof.

For example, in an embodiment of the invention, active ingredients can be blended with the hydrocolloid layer (b). Said active ingredient blended with the hydrocolloid layer (b) is then embossed, as discussed hereinabove, with a desired pattern of shapes to create the pockets (c). Thus, the active ingredients can be released at different rates depending upon the depth of the pockets(c). In an embodiment, different active ingredients can be added to each pocket. In other words, aliquots of each active, for example, Vitamin C and an antibiotic, can be inserted into a recently embossed pocket. The Vitamin C containing pocket can be of a different depth than the antibiotic containing pocket.

In an additional embodiment of the invention the wound dressing comprises:
(i.) a hydrophilic foam layer (a) ;
(ii) a hydrocolloid layer (b)wherein said hydrocolloid layer (b)is contoured with one or more pockets (c) ;
(iii) a film backing layer (d), wherein further, an active ingredient or mixtures thereof are contained within said one or more pockets ( c).

In another embodiment of the invention, the wound dressing comprises a hydrophilic foam layer ( a) to hydrocolloid layer (b) to said one or more pockets (c) to film backing layer (d) in a ratio of: 0.5- 8 mm (a) to 0.05 - 0.7mm (b) to 0.5 - 2mm ( c) to 0.01 - 0.05 mm (d ). In other words, the ratio of (a): (b): (c): (d) is : 0.5- 8 mm (a): 0.05 - 0.7mm (b): 0.5 - 2mm ( c) : 0.01 - 0.05 mm (d )

In another embodiment of the invention, the wound dressing comprises a hydrophilic foam layer ( a) to hydrocolloid layer (b) to said one or more pockets (c) to film backing layer (d) in a ratio of: 1-5 mm (a) to 0.05 - 0.2 mm (b) to 1.0 - 2.0 mm ( c) to 0.01 - 0.03 mm (d ). In other words, the ratio of (a): (b): (c): (d) is : 1-5 mm (a): 0.05 - 0.2mm (b): 1.0 - 2.0 mm ( c) : 0.01 - 0.03 mm (d )

According to another embodiment of the invention, the wound dressing comprises a hydrophilic foam layer ( a) to hydrocolloid layer (b) to said one or more pockets (c) to film backing layer (d) in a ratio of : 4mm (a) : 0.1mm (b) to 1.5 mm ( c) to 0.02mm (d). In other words, the ratio of (a): (b): (c): (d) is : 4 mm (a): 0.1mm (b): 1.5 mm ( c) : 0.02 mm (d )

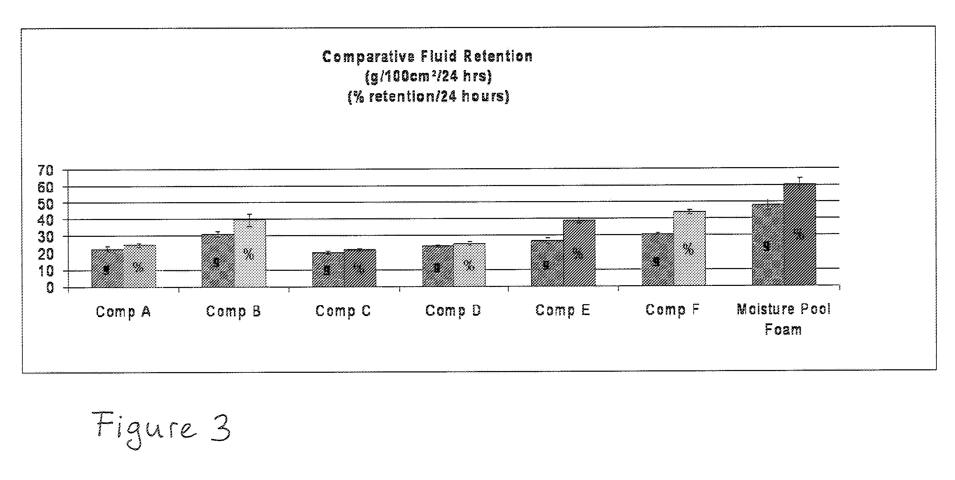

The absorptive capacity of the present invention, as compared to current absorptive dressings, is illustrated by the Comparative Fluid Retention Graph of Figure 3.

The Applicants' invention, labeled on the Graph as Moisture Pool Foam, clearly has lost less fluid than the currently available absorptive dressings. For example, the Applicants' invention retains 60% of the fluid over a 24 hour period. Also, the Applicants' invention retains 49g/ 100 square cm of fluid over a 24 hour period.

The current technologies, however, labeled as Comp A, B, C, D, E and F retained less fluid. For example, Comparative Example A ( labelled as Comp A) retains 24% of the fluid over a 24 hour period. Also, Comp A retains 22g/100 square cm over a 24 hour period.

The following non limiting examples, illustrate the practice of the present invention

### Example 1

The wound dressing of the present invention is constructed by placing a square hydrophilic foam pad on a hydrocolloid layer and then laminating a polyurethane film layer to the back of the hydrocolloid layer. The hydrocolloid pockets are then formed by heating and pressing the hydrocolloid layer (b) into the foam layer (a) to create desired pocket (c) shapes. This construction is made using standard medical converting equipment. The product is then placed in a hermetically sealed package and sterilized.

### Example 2 - Clinical Utility Example.

The wound dressing of the present invention is effective in the management of:
- Chronic and acute, moderate to heavy exudating, partial and full thickness wounds including superficial wounds
- 2nd degree burns
- Pressure ulcers, Stages II -IV

## Claims

1. A wound dressing comprising:
- a hydrophilic foam layer (a),
- a hydrocolloid layer (b), and
- a film backing layer (d),
**characterised in that** said hydrocolloid layer (b) is contoured with one or more pockets (c).

2. A wound dressing according to claim 1, wherein the proximal surface (5) of the hydrocolloid layer (b) is attached to the distal surface (2) of the hydrophilic foam layer (a), and wherein the one or more pockets (c) are formed as protrusions formed within the distal surface (3) of the hydrocolloid layer (b).

3. A wound dressing according to any of claims 1 or 2, wherein the thickness of the hydrophilic foam layer (a) is 0.5 - 8 mm, the thickness of the hydrocolloid layer is 0.05 - 0.7 mm, the depth (D') of the one or more pockets is 0.5 - 2 mm, and the thickness of the film backing layer (d) is 0.01 - 0.05 mm.

4. A wound dressing according to any of the preceding claims, wherein the thickness of the hydrophilic foam layer (a) is 1 - 5 mm, the thickness of the hydrocolloid layer is 0.05 - 0.2 mm, the depth (D') of the one or more pockets is 1 - 2 mm, and the thickness of the film backing layer (d) is 0.01 - 0.03 mm.

5. A wound dressing according to any of the preceding claims, wherein the thickness of the hydrophilic foam layer (a) is 4 mm, the thickness of the hydrocolloid layer is 0.1 mm, the depth (D') of the one or more pockets is 1.5 mm, and the thickness of the film backing layer (d) is 0.02 mm.

6. A wound dressing according to any of the preceding claims, wherein an active ingredient or mixtures thereof are contained within said one or more pockets.

7. A wound dressing according to any of the preceding claims, wherein the wound dressing consists of
- the hydrophilic foam layer (a),
- the hydrocolloid layer (b), and
- the film backing layer (d),
wherein said hydrocolloid layer (b) is contoured with one or more pockets (c).

8. A wound dressing according to any of the preceding claims, wherein at least 20% of the distal surface of the hydrocolloid layer is made up of pockets.

9. A wound dressing according to any of the preceding claims, wherein the hydrocolloid layer comprises carboxymethylcellulose, alginate and/or pectin.

10. A wound dressing according to any of the preceding claims, wherein the hydrophilic foam layer comprises polyurethane foam.

11. Method of manufacturing a wound dressing according to any one of the preceding claims, said method comprising the steps of:
- providing a hydrophilic foam layer (a) and a hydrocolloid layer (b),
- coating the hydrophilic foam layer (a) with the hydrocolloid layer (b) to form an absorbent layer, and
- embossing or stamping the absorbent layer with a pattern, thereby creating one or more pockets.

12. Method according to claim 11, wherein the coating step is carried out on the distal surface (2) of the hydrophilic foam layer (a).

13. Method according to claim 11 or 12, wherein the one or more pockets are formed as protrusions formed within the distal surface (3) of the hydrocolloid layer.

14. Method according to any of claims 11-13, wherein a film backing layer is attached to the distal surface (3) of the hydrocolloid layer prior to the embossing or stamping step.

15. Method according to any of claims 11-14, wherein the hydrocolloid layer comprises an active ingredient or a mixture thereof.
